(19) Europäisches Patentamt
European Patent Office
Office européen des brevets

(11) EP 3 227 254 B1

(12) # FASCICULE DE BREVET EUROPEEN

(45) Date de publication et mention de la délivrance du brevet:
**06.01.2021 Bulletin 2021/01**

(21) Numéro de dépôt: **15817483.9**

(22) Date de dépôt: **07.12.2015**

(51) Int Cl.:
***C07C 45/37*** *(2006.01)* ***C07C 319/18*** *(2006.01)*
***C07C 323/52*** *(2006.01)* ***C07C 59/76*** *(2006.01)*
***C07C 49/258*** *(2006.01)* ***C07C 49/24*** *(2006.01)*
***C07C 51/23*** *(2006.01)* ***C07C 45/39*** *(2006.01)*
***B01J 38/48*** *(2006.01)* ***B01J 37/08*** *(2006.01)*
***B01J 37/16*** *(2006.01)* ***B01J 38/02*** *(2006.01)*
***B01J 23/44*** *(2006.01)* ***B01J 27/28*** *(2006.01)*

(86) Numéro de dépôt international:
**PCT/FR2015/053348**

(87) Numéro de publication internationale:
**WO 2016/087807 (09.06.2016 Gazette 2016/23)**

(54) **OXYDATION CATALYTIQUE DU BUT-3-ÈNE-1,2-DIOL**
KATALYTISCHE OXIDATION VON BUT-3-EN-1,2-DIOL
CATALYTIC OXIDATION OF BUT-3-ENE-1,2-DIOL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.12.2014 FR 1461964**

(43) Date de publication de la demande:
**11.10.2017 Bulletin 2017/41**

(73) Titulaire: **Adisseo France S.A.S.**
**92160 Antony (FR)**

(72) Inventeurs:
- **REY, Patrick**
  **69003 Lyon (FR)**
- **BELLIERE-BACA, Virginie**
  **69390 Millery (FR)**
- **DUMEIGNIL, Franck**
  **59273 Fretin (FR)**
- **GRASSET, Fabien**
  **06130 Grasse (FR)**

(74) Mandataire: **Agasse, Stéphane et al**
**Cabinet GERMAIN & MAUREAU**
**B.P. 6153**
**69466 Lyon Cedex 06 (FR)**

(56) Documents cités:
**WO-A1-2013/018626** **FR-A1- 2 880 345**
**JP-A- S62 142 134** **JP-A- 2002 128 733**
**US-A- 5 155 263** **US-A1- 2004 204 597**

- **KOHSUKE MORI ET AL: "Hydroxyapatite-Supported Palladium Nanoclusters: A Highly Active Heterogeneous Catalyst for Selective Oxidation of Alcohols by Use of Molecular Oxygen", JOURNAL OF THE AMERICAM SOCIETY, vol. 126, no. 34, 1 September 2004 (2004-09-01), pages 10657-10666, XP055720523, US ISSN: 0002-7863, DOI: 10.1021/ja0488683**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne l'oxydation catalytique du but-3-ène-1,2-diol (BDO) et l'application de cette réaction à la préparation de composés biodisponibles de la méthionine et qui, selon l'invention, peuvent être utilisés en nutrition animale.

Ces composés sont l'acide 2-oxo-4-méthylthiobutanoïque (ci-après désigné par KMB), le 4-méthylthio-2-oxo-butanol (ci-après désigné par méthionol), le 2-oxo-4-méthylthiobutanal (ci-après désigné par méthional) et répondent aux structures ci-dessous,

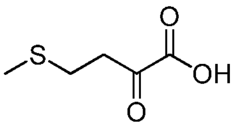
**KMB**

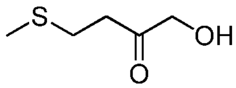
**Methionol**

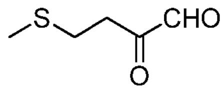
**Methional**

ainsi que leurs sels et leurs esters.

FR2880345A1 divulgue l'oxydation du but-3-ène-1,2-diol (BDO) en acide 2-oxo-but-3-ènoïque (CAV), en présence d'un catalyseur à base de palladium, platine, ruthénium, iridium et/ou rhodium, qui peut être activé par un promoteur métallique et qui peut être supporté par un support inerte choisi parmi l'alumine, la silice, les charbons actifs, le graphite, l'oxyde de titane, la zircone, le carbure de silicium, les oxydes mixtes à base de zircone et de cérium et le noir d'acétylène.

US2004/204597A1 enseigne que dans une réaction catalytique d'oxydation d'alcools, le catalyseur à base de ruthénium supporté sur de l'alumine est traité par une base pour augmenter les performances du catalyseur.

Les auteurs ont élaboré un procédé de préparation des composés ci-dessus, en deux étapes et dans des conditions permettant de limiter les temps de réaction tout en obtenant un bon rendement de conversion du BDO et d'améliorer au surplus la sélectivité de réaction.

La première étape fait intervenir l'oxydation du BDO en le céto-acide vinylique, l'acide 2-oxo-but-3-ènoïque (CAV), en le céto-alcool vinylique, l'hydroxybut-3-èn-2-one (CALV), et le céto-aldéhyde vinylique, le 2-oxo-but-3-èn-1-al (CADV), selon les équations suivantes :

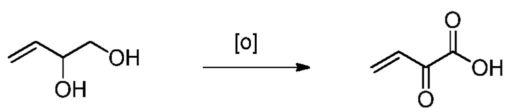

**BDO** **CAV**

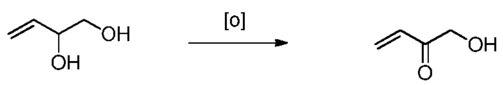

**BDO** **CALV**

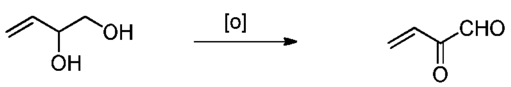

**BDO**

CADV

La deuxième étape consiste en l'addition de méthylmercaptan sur les céto-acide, -alcool et -aldéhyde précités selon les équations suivantes :

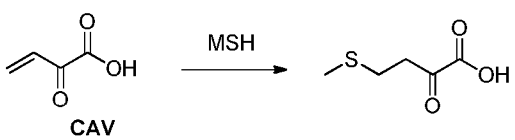

**CAV**

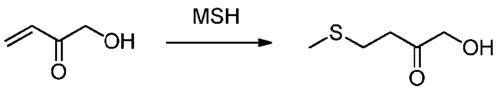


**CALV**

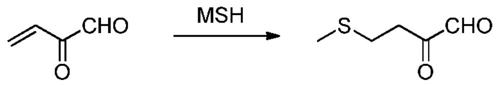


CADV

Selon un premier aspect de l'invention, elle concerne l'oxydation du BDO réalisée en présence d'un catalyseur, ledit catalyseur comprenant une phase active à base d'au moins un métal noble choisi parmi le palladium, l'or, l'argent, le platine, le rhodium, l'osmium, le ruthénium et l'iridium, et un support contenant des sites basiques. Selon une variante de l'invention, la phase active est à base de palladium. Lorsque la phase active est à base de palladium, elle peut consister en du palladium ; elle peut aussi consister en du palladium et un ou d'autres métaux nobles.

En plus des rendements de conversion du BDO et de la sélectivité de son oxydation, le procédé de l'invention permet de s'affranchir de tout ajustement du pH du milieu réactionnel et ainsi d'éviter d'ajouter une base dans le milieu réactionnel. Par rapport aux procédés connus mis en œuvre en présence de soude, ce qui entraîne la formation de sels nécessitant d'être neutralisés et générant des effluents aqueux salins peu prisés, le procédé de l'invention ne génère aucun sel et n'a donc pas recours à une étape de traitement ultérieur.

Avant d'exposer l'invention plus en détails, certains termes employés dans le texte sont ci-après définis.

Par support contenant des sites basiques, on entend selon l'invention un support contenant des sites de surface présentant un excédent électronique ; à titre d'exemple, ces sites sont constitués ou riches en ions tels que l'ion hydroxyle , l'ion $O^{2-}$; de préférence il est choisi parmi l'hydrotalcite (HT) et l'hydroxyapatite (HAP). Il peut aussi être choisi parmi des supports classiques ou basiques, après qu'ils sont modifiés pour les rendre basiques ou plus basiques. Ces modifications consistent notamment en le remplacement de certains ions du support, en une fixation de groupes basiques sur le support ou toute autre modification connue de l'homme du métier. A titre d'exemple, l'HP peut être modifiée en composés dits « hydrotalcite-like » (HTlc) comme suit : l'HP répond à la formule exacte $Mg_6Al_2(CO_3)(OH)_{16}\cdot 4H_2O$, et consiste en des couches de brucite $[Mg(OH)_2]$ cationiques entre lesquelles se trouvent des composés anioniques pour conduire à un matériau neutre. Il est possible d'effectuer des substitutions isomorphes au sein des couches de brucite pour remplacer les ions $Mg^{2+}$ et $Al^{3+}$. L'échange d'ions dans la couche cationique et/ou la couche anionique, induisant notamment un changement du ratio Mg/Al, permet d'ajuster la basicité du support. Par suite, les composés « hydrotalcite-like » (HTlc) possèdent la formule générale $[M(II)_{1-x}M'(III)_x(OH)_2]^{x+}$ $(A^{n-}{}_{x/n}).mH_2O$, où A est un anion, et M/M' des cations métalliques.

Le caractère basique d'un support selon l'invention est caractérisé par sa capacité à donner des doublets d'électrons ou à accepter des protons. Cette notion de support basique appartient aux connaissances générales de l'homme du métier. Il peut même recourir à des techniques classiques largement décrites dans la littérature. On peut noter, notamment les techniques de caractérisation des sites basiques de surface par spectroscopie vibrationnelle infrarouge après adsorption de molécules sondes acide (acétylène, méthanol, $CO_2$, propyne, ...), par thermodésorption de $CO_2$, par calorimétrie de $CO_2$, par réaction modèle (conversion du 2-méthyl-3-butyn-2-ol (MBOH), ...).

Un support de l'invention peut se présenter sous toute forme, en particulier toute forme géométrique sur laquelle la phase active du catalyseur peut être déposée. Il peut être poreux.

Les sels des composés obtenus selon l'un quelconque des procédés de l'invention sont de préférence des sels de cuivre, de calcium, de manganèse et de zinc.

D'autres aspects de l'invention, ainsi que des variantes préférentielles sont ci-après exposés. Bien entendu, dans le cadre de l'invention, les caractéristiques présentées peuvent être considérées seules ou combinaison.

Comme indiqué précédemment, la phase active consiste en un métal noble ou un mélange de métaux nobles. De préférence, le métal noble est le palladium, il peut aussi être en mélange avec un métal autre noble, avantageusement choisi parmi le platine et l'or.

La teneur en phase active d'un catalyseur selon l'invention va avantageusement de 0,005 à 50% en poids du métal ou des métaux constituant la phase active par rapport au poids du support sous sa forme oxyde.

Le support est choisi parmi les hydrotalcites (HT), les brucites, l'hydroxyapatite $Ca_{10}(PO_4)_6(OH)_2$, le phosphate de tricalcium $Ca_3(PO_4)_2$, l'hydrogénophosphate de calcium $CaHPO_4(0\text{-}2)H_2O$, le diphosphate de calcium $Ca_2P_2O_7$, le phosphate d'octacalcium $Ca_8H_2(PO_4)_6.5H_2O$, le phosphate de tétracalcium $Ca_4(PO_4)_2O$, les phosphate de calcium amorphe $Ca_3(PO_4)_2.nH_2O$, les phosphates, diphosphates, et hydrogénophosphates de calcium, de césium, de lithium, de rubidium, de strontium, de potassium, de magnésium, de baryum, de cérium, de lanthane, d'aluminium, de zinc et/ou de cuivre, et parmi tous mélanges de ceux-ci.

Avantageusement, le support est choisi parmi les composés répondant aux formules A, B et C suivantes, et leurs mélanges :

Formule (A), $M_{\alpha}[M_{(1-b)}La_{b}]A^{z-}]_{c}$ où

M est choisi parmi le groupe composé de $Mg^{2+}$, $Ca^{2+}$, $Sr^{2+}$, $Ba^{2+}$, $Ra^{2+}$, et leurs combinaisons,
$A^{z-}$ est un anion monovalent ou divalent choisi parmi le groupe composé des carbonate ($CO_3^{2-}$, où la charge « z » est données par z=2), oxyde ($O^{2-}$, où z=2), hydroxyde ($OH^-$, où z=1), et bicarbonate ($HCO_3^-$, où z=1) ou un mélange ($A^{z'-}{}_xA^{z''-}{}_y$) d'anions divalent et monovalent avec $A^{z'-}$et $A^{z''-}$ des anions différents, avec $A^{z-}$ est $A^{z'}{}_xA^{z''-}{}_y$ avec une charge z donnée par z=x(z')+y(z") et x+y=1
$\alpha$ varie de 0,01 à 0,4
b varie de 0,0011 à 0,11

$$c=(2\alpha/z)+[3(1-b)/z]+(3b/z)$$

Formule (B) : $(M_dM'_eM''_fM'''_g)_5(PO_4)_3(OH)$ où

M est $Mg^{2+}$ ; M' est $Ca^{2+}$ ; M" est $Sr^{2+}$ ; M'" est $Ba^{2+}$
d varie de 0 à 1
e varie de 0 à 0,5
f varie de 0 à 1
g varie de 0 à 1
d+e+f+g=1

Formule (C) $(M_dM'_eM''_fM'''_g)_3(PO_4)_2$ où

M est $Mg^{2+}$ ; M' est $Ca^{2+}$ ; M" est $Sr^{2+}$ ; M'" est $Ba^{2+}$
d varie de 0 à 1
e varie de 0 à 1
f varie de 0 à 1
g varie de 0 à 1
d+e+f+g=1.

Selon une forme de mise en œuvre de l'invention, le catalyseur comprend, en plus de la phase active et du support, un promoteur. Celui-ci est de préférence choisi parmi le bismuth, le plomb, l'antimoine, l'étain, le niobium, le tellure, l'indium, le gallium, le zinc, le cuivre, le nickel, le cobalt, l'argent, le tungstène, le molybdène, le zirconium, le vanadium, le chrome, le manganèse, le fer, le cérium, le praséodyme, le samarium, le titane et leurs mélanges.

Selon d'autres aspects de l'invention, les conditions catalytiques de l'oxydation du BDO permettent d'obtenir majoritairement l'un des céto-acide, -alcool et -aldéhyde précités, voire de n'obtenir que l'un des trois.

Ainsi, dans une forme de réalisation de l'invention, le composé (I) est le céto-acide vinylique (CAV).

Dans une autre forme de réalisation de l'invention, l'oxydation est réalisée en présence d'un catalyseur dont la phase active est choisie parmi le palladium et les mélanges de palladium et de platine et le support à sites basiques est choisi parmi l'hydroxyapatite et l'hydrotalcite, et le composé (I) est le céto-alcool vinylique (CALV).

Dans encore une autre forme de réalisation de l'invention, le composé (I) est le céto-aldéhyde vinylique (CADV).

L'invention concerne aussi les applications d'un procédé de synthèse tel que défini ci-dessus.

Ainsi, elle concerne la synthèse d'au moins un composé de formule (II) suivante ou l'un de ses sels,

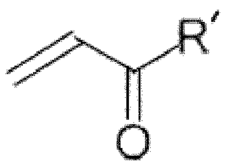

Formule (II)

où R' représente un groupe COOR1 ou $CH_2OR2$ pour lequel R1 et R2, identiques ou différents, représentent un groupe choisi parmi les groupes alkyle, linéaires ou ramifiés, ayant de 1 à 12 atomes de carbone, et les groupes cycloalkyle ayant de 3 à 12 atomes de carbone, synthèse dans laquelle :

on soumet le but-3-ène-1,2-diol (BDO) à une oxydation en présence d'un catalyseur, ledit catalyseur comprenant une phase active à base d'au moins un métal noble choisi parmi le palladium, l'or, l'argent, le platine, le rhodium, l'osmium, le ruthénium et l'iridium, et un support contenant des sites basiques, défini selon la revendication 1 pour obtenir un composé de formule (I) suivante,

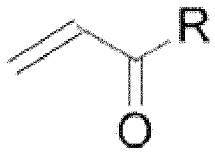

Formule (I)

où R représente un groupe COOH, $CH_2OH$ ou CHO, dans les conditions définies dans la revendication 1, et on réalise l'estérification ou l'éthérification du composé de formule (I) pour obtenir le composé de formule (II).

Cette synthèse donne alors accès aux dérivés esters et alkoxylés des composés de Formule (I), qui sont aussi des précurseurs de composés biodisponibles de la méthionine d'intérêt.

L'invention concerne également un procédé de synthèse d'au moins un composé de formule (III) suivante ou l'un de ses sels,

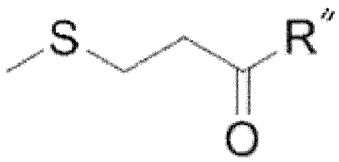

Formule (III)

Où R" représente un groupe COOH, COOR1, $CH_2OH$, $CH_2OR2$ ou CHO pour lesquels R1 et R2, identiques ou différents, représentent un groupe alkyle, linéaire ou ramifié, ayant de 1 à 12 atomes de carbone, et les groupes cycloalkyle ayant de 3 à 12 atomes de carbone, synthèse dans laquelle :

on soumet le but-3-ène-1,2-diol (BDO) à une oxydation en présence d'un catalyseur pour obtenir au moins un composé de formule (I) suivante ou l'un de ses sels,

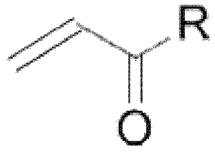

Formule (I)

où R représente un groupe COOH, $CH_2OH$ ou CHO, selon le procédé décrit précédemment,

si R" représente un groupe COOR1 ou $CH_2OR2$, on réalise une estérification ou une éthérification du composé de formule (I) pour obtenir au moins un composé de formule (II) suivante ou l'un de ses sels,

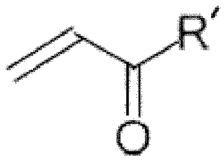

Formule (II)

où R' représente un groupe COOR1 ou $CH_2OR2$ pour lequel R1 et R2, identiques ou différents, représentent un groupe choisi parmi les groupes alkyle, linéaires ou ramifiés, ayant de 1 à 12 atomes de carbone, et les groupes cycloalkyle ayant de 3 à 12 atomes de carbone,
Et, on fait réagir ledit composé (I) ou ledit composé (II) ou lesdits un de leurs sels, avec le méthylmercaptan pour obtenir ledit composé (III) ou ledit un de ses sels, au moins.

L'addition du méthylmercaptan est effectuée dans des conditions bien connues de l'homme du métier. Elle peut être réalisée en l'absence ou en présence d'un solvant et d'un catalyseur homogène basique.

Selon une variante du procédé, l'oxydation est réalisée dans les conditions permettant d'obtenir le CAV, et le composé (III) est l'acide 2-oxo-4-méthylthiobutyrique.

Selon une autre variante du procédé, l'oxydation est réalisée dans les conditions permettant d'obtenir le CALV et le composé (III) est le 1-hydroxy-4-méthylthiobuta n-2-one.

Selon encore une autre variante du procédé, l'oxydation est réalisée dans les conditions permettant d'obtenir le CADV et le composé (III) est le 2-oxo-4-méthylthiobutanal.

L'oxydation du BDO selon l'invention est ci-après décrite plus en détails.

Le BDO peut se présenter sous sa forme liquide, à l'état purifié ou non, ou bien sous une forme de solution aqueuse brute, c'est-à-dire de moindre pureté, par exemple résultant de sa préparation. Selon une variante de l'invention, le BDO est en solution aqueuse en une concentration allant de 1 à 70% en poids par rapport au poids de la solution.

Le BDO peut être obtenu à partir du butadiène par monoépoxydation de celui-ci en 3,4-époxy-1-butène qui est converti en BDO (II) par ouverture chimique de la fonction époxyde avantageusement acido-catalysée et en milieu aqueux.

Quelle que soit sa forme, le diol est directement utilisable pour la réaction d'oxydation catalytique en acide-2-oxo-but-3-ènoïque (CAV), en hydroxybut-3-èn-2-one (CALV) ou en 2-oxobut-3-èn-1-al (CADV). De manière avantageuse, la solution aqueuse qui résulte de l'ouverture de l'époxyde ci-dessus sera directement engagée à l'étape d'oxydation du BDO.

Le catalyseur d'oxydation du BDO à base de métaux nobles peut comprendre au moins un promoteur choisi parmi le bismuth, le plomb, l'antimoine, l'étain, le niobium, le tellure, l'indium, le gallium, le zinc, le cuivre, le nickel, le cobalt, l'or, l'argent, le tungstène, le molybdène, le rhénium, le vanadium, le chrome, le manganèse, le fer et leurs mélanges.

La teneur en promoteur est comprise entre 0,005 et 500%, de préférence entre 0,005 et 100%, en poids par rapport au poids du support sous sa forme oxyde. Le dépôt du promoteur sur le support catalytique est avantageusement réalisé par imprégnation.

La préparation du catalyseur est réalisée par imprégnation en maintenant sous agitation le mélange support de catalyseur et la solution contenant le ou les métaux nobles, pendant une durée variant d'au moins quelques secondes à quelques heures, généralement comprise entre 2 et 16 heures. Le catalyseur est ensuite séché puis optionnellement imprégné par la solution du promoteur. Cette opération précède une optionnelle étape de calcination du catalyseur qui s'effectue sous air statique à une température comprise entre 20 et 800°C. Une réduction du catalyseur peut être effectuée à une température comprise entre 20 et 400°C, à l'aide d'agents chimiques réducteurs du type formol, formiate de sodium, borohydrure de sodium, hydrogène, acide hypophosphoreux, hydrazine, glucose ou autres sucres réducteurs.

Une alternative de préparation du catalyseur est de réaliser une première imprégnation du promoteur suivie d'une seconde étape d'imprégnation du ou des métaux nobles. Une réduction du catalyseur est ensuite opérée.

Une autre alternative de préparation du catalyseur est de réaliser en une seule imprégnation du ou des métaux nobles et du promoteur. Une réduction du catalyseur est ensuite opérée.

Les conditions réactionnelles d'une oxydation du BDO selon l'invention sont ci-dessous décrites plus en détails et sont illustrées dans les exemples qui suivent :
on introduit dans un réacteur muni d'un dispositif d'agitation, une solution aqueuse du BDO, la concentration en BDO

étant de préférence comprise entre 1 et 70% en poids. La limite inférieure de la concentration en diol est dictée par un souci de rentabilité du procédé et sa limite supérieure tient compte de la solubilité de l'oxygène dans les milieux considérés et du risque de cristallisation du dérivé formé pendant la réaction ;

- on disperse dans cette solution une quantité d'un catalyseur, de préférence supporté et activé tel que décrit ci-dessus ;
- on amorce la réaction d'oxydation par l'apport simultané d'un balayage d'un gaz contenant de l'oxygène comme l'air.

La température de réaction se situe généralement entre 10°C et 95°C, de préférence entre 20 et 95°C, voire entre 25°C et 70°C, pour un temps de réaction compris entre 20 minutes et 15 heures.

Le procédé selon l'invention permet d'atteindre des sélectivités très intéressantes excédant 90%. Ces performances ne sont pas altérées par un nombre important de recyclage et/ou de réactivation du catalyseur d'oxydation mis en œuvre conformément à la présente invention. Les catalyseurs employés possèdent en effet une durée de vie appréciable et sont facilement régénérés *in situ* par dépôt d'une nouvelle charge de promoteur ou par réduction *in situ* du catalyseur désactivé.

Cette première étape d'oxydation est, avantageusement, pratiquée en solvant aqueux. Un solvant organique ou un mélange de solvants organiques peuvent aussi être employés. Un milieu hydro-organique peut aussi s'avérer profitable.

Le solvant organique, constituant le milieu dans lequel s'effectue la réaction d'oxydation du BDO, est choisi parmi tout solvant au moins partiel dudit diol (BDO), inerte dans les conditions opératoires, en particulier le ou les solvants sont avantageusement choisis parmi les hydrocarbures aliphatiques, cycloaliphatiques ou aromatiques et en particulier parmi :

- les esters d'alkyle ou alkényle d'acides carboxyliques aliphatiques; les éthers aliphatiques, aromatiques ou cycliques; les nitriles aliphatiques; cycloaliphatiques ou aromatiques; les cétones aliphatiques, cycloaliphatiques ou aromatiques. A titre d'exemples non limitatifs, on peut citer :
    - des hydrocarbures comme le n-hexane, n-heptane, n-octane, n-nonane, le benzène, le styrène, l'éthylbenzène, le toluène, le métaxylène, l'isopropylbenzène, le cyclohexane, le méthyl-4-pentène-2 ;
    - des esters comme le formiate d'éthyle, le formiate de butyle, le formiate d'isobutyle, l'acétate d'éthyle, l'acétate d'allyle, l'acétate de propyle, l'acétate de butyle, l'acétate d'hexyle, le propionate d'éthyle, le propionate de vinyle, l'acrylate d'éthyle, le butyrate de butyle, l'isobutyrate de méthyle ; le butyrate de méthyle ;
    - des éthers comme l'éthoxy-1-butène-1 cis, l'éthoxy-1-butène-1-trans, l'oxyde de dibutyle, l'isopropoxy-1-butane, le diméthoxy-1,1-éthane, diéthoxy-1,1-éthane, diméthoxy-1,1-propane, l'éthoxy-1-butane, l'oxyde de diisopropyle, l'éthoxy-1-hexane, l'éthoxy-2-propane, le méthoxy-1-butadiène-1,3, l'oxyde de butyle et de vinyle, le furane, le diméthyl-2,5-furane ;
    - des nitriles comme le butyronitrile, l'acétonitrile, l'acrylonitrile, le propionitrile, le tétrahydrobenzonitrile ;
    - des cétones telles que la cyclopentanone, la dipropylcétone, l'heptanone, la méthylisopropylcétone, la méthyl-5-hexanone-2, la pentanone-2, la méthyl-4 pentène-3-one-2.

L'oxygène utilisé pour amorcer la réaction d'oxydation peut être de l'oxygène moléculaire, de l'air, de l'air enrichi, ou appauvri en oxygène ou tout autre mélange de l'oxygène avec un gaz inerte.

La pression totale sous laquelle s'effectue la réaction peut être supérieure, égale ou inférieure à la pression atmosphérique ; elle est généralement comprise entre 0,5 et 10 bars. La pression partielle d'oxygène est de préférence, comprise entre 0,05 bar et 5 bars. L'oxydation du BDO peut être effectuée soit en maintenant une pression constante, soit en faisant circuler l'oxygène ou le gaz en contenant, dans l'appareil où s'effectue la réaction, soit encore en faisant barboter l'oxygène ou le gaz en contenant, dans le mélange réactionnel.

L'appareillage dans lequel le procédé selon l'invention est mis en œuvre peut bien entendu ne pas être spécifique audit procédé.

L'étape de condensation d'un composé (I) de l'invention avec le méthylmercaptan pour obtenir un composé (II) conformément à l'invention est ci-après exposée en détails.

Selon cette étape, une mole de méthylmercaptan (MeSH) sous sa forme gazeuse ou liquide et une mole de CAV ou de CALV ou de CADV précédemment préparés sont condensés selon le schéma réactionnel ci-après:

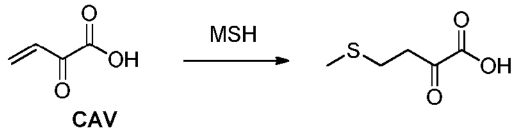


**CAV**

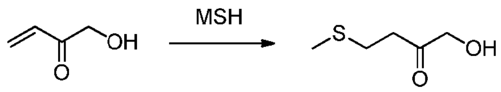


**CALV**

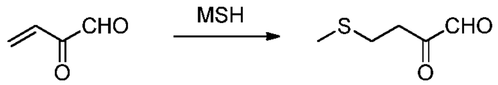


Le domaine de la présente invention est celui de la fabrication de KMB, du méthionol, du méthional et leurs mélanges en tant que produit final, ou produit intermédiaire. La réactivité des thiols est, à bien des égards, similaire à celle des alcools. Ils peuvent, suivant les conditions catalytiques mises en œuvre, s'additionner sur les aldéhydes $\alpha,\beta$-insaturés, les cétones $\alpha,\beta$-insaturés et les acides $\alpha,\beta$-insaturés, en position 1,2 conduisant au mono-hémithioacétal ou en position 1,4 conduisant à l'aldéhyde 3-alkylthiopropionique. Par analogie structurale, les cétoacides vinyliques s'inscrivent pleinement dans la catégorie des oléfines activées.

Deux voies catalytiques sont traditionnellement préconisées pour additionner sélectivement et efficacement les thiols en position 4 sur les dérivés carbonylés $\alpha,\beta$-insaturés. La première est une addition ionique catalysée par les bases. La seconde est une addition radicalaire initiée par les composés azoïques ou peroxydes. Cependant, ce mode d'initiation conduit généralement à des polymères le plus souvent indésirables.

L'addition 1,4 de type Michael des thiols sur les cétones $\alpha$-$\beta$-insaturées est de préférence utilisée.

Le produit brut de départ comprenant le KMB, le méthionol, le méthional, salifié ou non, peut subir un premier traitement permettant d'éliminer les impuretés coproduites lors de l'oxydation du BDO. Ce produit brut peut aussi être soumis à un dégazage. L'excès du BDO correspondant au diol qui n'a pas réagi, peut être avantageusement recyclé à l'étape d'oxydation, par exemple, par distillation ou extraction. La solution aqueuse de CAV, de CALV ou de CADV peut, éventuellement, subir une concentration préalable à la mise en contact avec le méthylmercaptan gazeux ou liquide. Elle est ensuite mise en contact avec du méthylmercaptan gazeux ou liquide pour conduire au KMB, ou méthionol ou méthional.

Cette étape peut éventuellement, être effectuée en présence d'un catalyseur ou d'un mélange de catalyseurs basiques. Des catalyseurs basiques appropriés sont, par exemple, les amines aliphatiques telles que la méthylamine, l'éthylamine, la propylamine, la butylamine, la pentylamine, l'hexylamine, l'heptylamine, l'octylamine, l'isopropylamine, la triallylamine; les amines aromatiques comme l'aniline, la benzylamine, la pyridine ; l'hexaméthylènetétramine, la triéthylamine, la diisopropyléthylamine, diazabicylo[2,2,2]octane, la N,N-diméthylbenzylamine, la N-méthyldiphénylamine, la N-éthyl-3,3'-diphényldipropylamine, une N-alkylmorpholine, telle que la N-méthylmorpholine, ou bien le triton B. Ces amines étant éventuellement combinées à un acide organique ou minéral ; celui-ci est de préférence choisi parmi l'acide formique, l'acide acétique, l'acide propanoïque et l'acide butanoïque, l'acide phosphorique et l'acide sulfurique.

L'addition du méthylmercaptan est avantageusement acido-baso catalysée, par exemple au moyen d'un catalyseur consistant en une combinaison d'un acide organique ou minéral et une base organique ou minérale. L'acide acétique est préférentiellement utilisé.

A l'échelle industrielle, le méthylmercaptan, liquide ou gazeux, est acheminé dans un réacteur contenant la solution aqueuse, préalablement concentrée ou pas, dégazée ou non, du KMB, ou méthionol ou méthional.

La condensation entre le CAV, le CALV, le CADV et le méthylmercaptan peut être conduite en batch ou en continu. Ils sont introduits simultanément ou alternativement, en respectant le rapport stœchiométrique. On peut cependant envisager de travailler en défaut ou excès de méthylmercaptan, selon la réaction poursuivie.

La réaction peut se pratiquer par introduction continue entre la solution aqueuse du composé (I) et le méthylmercaptan gazeux dans un réacteur gaz/liquide. Dans ce cas, le méthylmercaptan pourra être ajouté à co- ou contre-courant. Alternativement, la réaction peut se pratiquer par introduction continue de la solution aqueuse du ou des dérivés insaturés et du méthylmercaptan liquide dans un réacteur batch ou piston. La température de réaction ne devra pas excéder 80°C.

Les catalyseurs de condensation entre les dérivés insaturés et le méthylmercaptan sont généralement choisis en fonction de plusieurs critères :

- la conversion et rendement en acide-2-oxo-but-3-énoïque (CAV), ou hydroxybut-3-én-2-one (CALV) ou 2-oxo-but-3-én-1-al (CADV) ;

- la cinétique réactionnelle ;
- la sélectivité et tendance à coproduire des impuretés indésirables qui sont, habituellement, des espèces de haut poids moléculaire, issues de polymérisations parasites pendant la synthèse mais aussi durant le stockage du produit recherché ;
- la faculté à stabiliser le produit durant son stockage prolongé.

L'appareillage dans lequel le procédé selon l'invention est mis en œuvre n'est pas spécifique audit procédé.
Les exemples qui suivent et les figures auxquelles ils font référence illustrent la synthèse de catalyseurs et leur mise en œuvre dans un procédé d'oxydation selon l'invention.

**Exemples**

**Exemple 1 : Préparation de catalyseurs d'oxydation pour la mise en œuvre d'un procédé selon l'invention**

Dans les préparations ci-dessous, la teneur en phase est active est exprimée en poids du ou des métaux la constituant par rapport au poids du support sous forme oxyde.

Préparation de 2%Pd/HAP (catalyseur 4)

A 4 mL d'une solution aqueuse de $H_2PdCl_4$ contenant 20 mg de Pd a été ajouté à température ambiante sous agitation vigoureuse 0,98 g de d'hydroxyapatite (HAP, $Ca_{10}(PO_4)_6(OH)_2$), comme décrit dans la littérature [K. Mori, T. Hara, T. Mizugaki, K. Ebitani, K. Kaneda, J. Am. Chem. Soc. (2004) 126 (34):10657-10666]).

Préparation de 2%PdPt/HAP 50-50 (catalyseur 5)

La procédure pour la préparation du catalyseur 4 a été utilisée mais en partant de 4 mL d'une solution aqueuse de $H_2PdCl_4$ et $H_2PtCl_6$ contenant 10 mg de Pd et 10 mg de Pt.

Préparation de 4%Pdl%Pt/HAP (catalyseur 8)

A 10 mL d'une solution aqueuse de $H_2PtCl_6$ et $H_2PdCl_4$ contenant 10 mg of Pt et 40 mg de Pd a été ajouté à température ambiante 0,95 g de HAP. La solution a été agitée à 40°C pendant 2 h puis évaporée à sec. Le solide résultant a été broyé et calciné à 400°C pendant 3 h.

Préparation de 5%Pd/HAP (catalyseur 9)

La procédure pour la préparation du catalyseur 4 a été utilisée mais en partant de 10 mL d'une solution aqueuse de $H_2PdCl_4$ contenant 50 mg de Pd et en utilisant 0,95 g de HAP.

Préparation de 2%Pd/HAP (catalyseur 10)

La procédure pour la préparation du catalyseur 8 a été utilisée mais en partant de 20 mL d'une solution aqueuse de $H_2PdCl_4$ contenant 100 mg de Pd et en utilisant 4,90 g de HAP.

Préparation de 4%Pdl%Pt/HT (catalyseur 11)

La procédure pour la préparation du catalyseur 8 a été utilisée mais en utilisant 0,95 g de Mg-Al hydrotalcite (HT, Mg/Al = 5) préparée selon une procédure de la littérature [N. K. Gupta, S. Nishimura, A. Takagaki, K. Ebitani, Green Chem. (2011) 13:824-827].

Préparation de 2%Pd/HAP sans calcination (catalyseur 12)

La procédure pour la préparation du catalyseur 8 a été utilisée mais après évaporation, au lieu d'être calciné, le catalyseur a été séché dans une étuve à 70°C pendant 3 jours.

**Exemple 2 : Oxydation du but-3-ène-1,2-diol (BDO) avec les catalyseurs de l'exemple 1, selon l'invention**

Oxydation du BDO avec le catalyseur 4

Le catalyseur 4 (30 mg) a été introduit dans un tube en verre, mis sous oxygène via 3 cycles vide/oxygène puis une solution de BDO (0,1 M, 3 mL) dans l'eau a été introduite. Le mélange a été agité (600 tour/minute) à 50°C sous $O_2$ (1 atm) pendant 5 h. Après retour à température ambiante, le catalyseur a été séparé par filtration et la solution a été analysée par HPLC (détecteurs IR et UV) et GC/GC-MS pour déterminer la conversion et la sélectivité des produits.
La conversion du BDO est de 73%. La sélectivité pour le CALV est de 87%.

Oxydation du BDO avec le catalyseur 5

La procédure de test du catalyseur 4 a été répétée mais en utilisant le catalyseur 5 (30 mg).
La conversion du BDO est de 55%. La sélectivité pour le CALV est de 89%.

Oxydation du BDO avec le catalyseur 8

La procédure de test du catalyseur 4 a été répétée mais en utilisant le catalyseur 8 (30 mg).
La conversion du BDO est de 92%. La sélectivité pour le CALV est de 85%.

Oxydation du BDO avec le catalyseur 9

La procédure de test du catalyseur 4 a été répétée mais en utilisant le catalyseur 9 (30 mg).
La conversion du BDO est de 88%. La sélectivité pour le CALV est de 88%.

Oxydation du BDO avec le catalyseur 10

La procédure de test du catalyseur 4 a été répétée mais en utilisant le catalyseur 10 (30 mg).
La conversion du BDO est de 66%. La sélectivité pour le CALV est de 87%.

Oxydation du BDO avec le catalyseur 11

La procédure de test du catalyseur 4 a été répétée mais en utilisant le catalyseur 11 (30 mg).
La conversion du BDO est de 83%.

Oxydation du BDO avec le catalyseur 12

La procédure de test du catalyseur 4 a été répétée mais en utilisant le catalyseur 12 (30 mg).
La conversion du BDO est de 83%. La sélectivité pour le CALV est de 88%.

**Exemple 3 : Oxydation du but-3-ène-1,2-diol (BDO) avec des catalyseurs de l'exemple 1, selon l'invention, recyclés**

Oxydation du BDO avec le catalyseur 4 (2%Pd/HAP)

Elle est réalisée dans les conditions décrites à l'exemple 3, avec le catalyseur 4. A la fin de la réaction, le catalyseur est séparé du milieu réactionnel par centrifugation, lavé 5 fois à l'eau déminéralisée et séché une nuit à 70°C.
La conversion du BDO et la sélectivité du procédé pour le CALV sont illustrées à la Figure 1, pour chaque essai, la première colonne représentant le pourcentage de conversion du BDO, la seconde la sélectivité en CALV.

Oxydation du BDO avec le catalyseur 5 (2%PdPt/HAP)

Elle est réalisée dans les conditions décrites à l'exemple 3, avec le catalyseur 5. A la fin de la réaction, le catalyseur est séparé du milieu réactionnel par centrifugation, lavé 5 fois à l'eau déminéralisée et séché une nuit à 70°C.
La conversion du BDO et la sélectivité du procédé pour le CALV sont illustrées à la Figure 2, pour chaque essai, la première colonne représentant le pourcentage de conversion du BDO, la seconde la sélectivité en CALV.
Il ressort de cet exemple que les catalyseurs de l'invention sont recyclables. Leur recyclage n'affecte en rien la sélectivité de la réaction d'oxydation et n'abaisse que très modérément le taux de conversion du BDO.

## Revendications

**1.** Procédé de synthèse d'au moins un composé de formule (I) suivante ou l'un de ses sels,

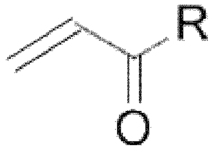

Formule (I)

où R représente un groupe COOH, $CH_2OH$ ou CHO,

**Caractérisé en ce que**

on soumet le but-3-ène-1,2-diol (BDO) à une oxydation en présence d'un catalyseur, ledit catalyseur comprenant une phase active à base d'au moins un métal noble choisi parmi le palladium, l'or, l'argent, le platine, le rhodium, l'osmium, le ruthénium et l'iridium, et un support contenant des sites basiques et choisi parmi les hydrotalcites (HT), les brucites, l'hydroxyapatite $Ca_{10}(PO_4)_6(OH)_2$, le phosphate de tricalcium $Ca_3(PO_4)_2$, l'hydrogénophosphate de calcium $CaHPO_4(0\text{-}2)H_2O$, le diphosphate de calcium $Ca_2P_2O_7$, le phosphate d'octacalcium $Ca_8H_2(PO_4)_6.5H_2O$, le phosphate de tétracalcium $Ca_4(PO_4)_2O$, les phosphates de calcium amorphes $Ca_3(PO_4)_2.nH_2O$, les phosphates, diphosphates, et hydrogénophosphates de calcium, de césium, de lithium, de rubidium, de potassium, de magnésium, de baryum, de cérium, de lanthane, d'aluminium, de zinc, de cuivre, et leurs mélanges.

**2.** Procédé selon la revendication 1, **caractérisé en ce que** la phase active consiste en du palladium ou en un mélange de palladium et d'au moins un métal noble choisi parmi le platine et l'or.

**3.** Procédé selon la revendication 1 ou 2, **caractérisé en ce que** la phase active consiste en un métal noble ou un mélange de métaux nobles en une teneur allant de 0,005 à 50% en poids par rapport au poids du support sous forme oxyde.

**4.** Procédé selon la revendication 1, **caractérisé en ce que** le support est choisi parmi les composés répondant aux formules A, B et C suivantes, et leurs mélanges :

Formule (A), $M_{\alpha}[Al_{(1-b)}La_{b}]A^{z-}]_{c}$ où

M est choisi parmi le groupe composé de $Mg^{2+}$, $Ca^{2+}$, $Sr^{2+}$, $Ba^{2+}$, $Ra^{2+}$, et leurs combinaisons,

$A^{z-}$ est un anion monovalent ou divalent choisi parmi le groupe composé des carbonate ($CO_3^{2-}$, où la charge « z » est données par z=2), oxyde ($O^{2-}$, où z=2), hydroxyde (OH-, où z=1), et bicarbonate ($HCO_3^-$, où z=1) ou un mélange ($A^{z'-}{}_xA^{z''-}{}_y$) d'anions divalent et monovalent avec $A^{z'-}$et $A^{z''-}$ des anions différents, avec $A^{z-}$ est $A^{z'-}{}_xA^{z''-}{}_y$ avec une charge z donnée par z=x(z')+y(z") et x+y=1

$\alpha$ varie de 0,01 à 0,4

b varie de 0,0011 à 0,11

$$c=(2\alpha/z)+[3(1-b)/z]+(3b/z)$$

Formule (B) : $(M_dM'_eM''_fM'''_g)_5(PO_4)_3(OH)$ où

M est $Mg^{2+}$ ; M' est $Ca^{2+}$ ; M" est $Sr^{2+}$ ; M'" est $Ba^{2+}$

d varie de 0 à 1

e varie de 0 à 0,5

f varie de 0 à 1

g varie de 0 à 1

d+e+f+g=1

Formule (C) $(M_dM'_eM''_fM'''_g)_3(PO_4)_2$ où

M est $Mg^{2+}$ ; M' est $Ca^{2+}$ ; M" est $Sr^{2+}$ ; M'" est $Ba^{2+}$
d varie de 0 à 1
e varie de 0 à 1
f varie de 0 à 1
g varie de 0 à 1
d+e+f+g=1.

**5.** Procédé selon l'une quelconque des revendications 1 à 4, **caractérisé en ce que** le catalyseur comprend un promoteur choisi parmi le bismuth, le plomb, l'antimoine, l'étain, le niobium, le tellure, l'indium, le gallium, le zinc, le cuivre, le nickel, le cobalt, l'argent, le tungstène, le molybdène, le zirconium, le vanadium, le chrome, le manganèse, le fer, le cérium, le praséodyme, le samarium, le titane et leurs mélanges.

**6.** Procédé selon l'une quelconque des revendications 1 à 5, **caractérisé en ce que** la teneur du promoteur du catalyseur va de 0,005 et 500%, de préférence entre 0,005 et 100%, en poids par rapport au poids du support sous forme oxyde.

**7.** Procédé selon l'une quelconque des revendications 1 à 6, **caractérisé en ce que** le BDO est en solution aqueuse, en une concentration allant de 1 à 70% en poids par rapport au poids de la solution.

**8.** Procédé selon l'une quelconque des revendications 1 à 7, **caractérisé en ce que** l'oxydation est réalisée en présence d'un catalyseur dont la phase active est choisie parmi le palladium et les mélanges de palladium et de platine et le support à sites basiques est choisi parmi l'hydroxyapatite et l'hydrotalcite, et le composé (I) est le céto-alcool vinylique (CALV).

**9.** Procédé de synthèse d'au moins un composé de formule (II) suivante ou l'un de ses sels,

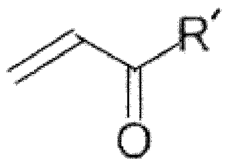

Formule (II)

où R' représente un groupe COOR1 ou $CH_2OR2$ pour lequel R1 et R2, identiques ou différents, représentent un groupe choisi parmi les groupes alkyle, linéaires ou ramifiés, ayant de 1 à 12 atomes de carbone, et les groupes cycloalkyle ayant de 3 à 12 atomes de carbone,

**caractérisé en ce que**

on soumet le but-3-ène-1,2-diol (BDO) à une oxydation en présence d'un catalyseur, selon l'une quelconque des revendications 1 à 8, pour obtenir un composé de formule (I) suivante,

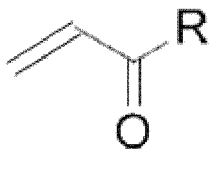

Formule (I)

où R représente un groupe COOH, $CH_2OH$ ou CHO, selon un procédé défini à l'une quelconque des revendications 1 à 8, et

et on réalise l'estérification ou l'éthérification du composé de formule (I) pour obtenir le composé de formule (II).

**10.** Procédé de synthèse d'au moins un composé de formule (III) suivante ou l'un de ses sels,

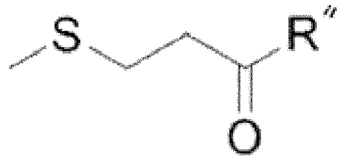

Formule (III)

où R" représente un groupe COOH, COOR1, $CH_2OH$, $CH_2OR2$ ou CHO pour lesquels R1 et R2, identiques ou différents, représentent un groupe alkyle, linéaire ou ramifié, ayant de 1 à 12 atomes de carbone, et les groupes cycloalkyle ayant de 3 à 12 atomes de carbone,
**caractérisé en ce que**
on soumet le but-3-ène-1,2-diol (BDO) à une oxydation en présence d'un catalyseur pour obtenir au moins un composé de formule (I) suivante ou l'un de ses sels,

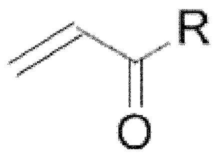

Formule (I)

où R représente un groupe COOH, $CH_2OH$ ou CHO, selon un procédé défini à l'une quelconque des revendications 1 à 8,
si R" représente un groupe COOR1 ou $CH_2OR2$, on réalise l'estérification ou l'éthérification du composé de formule (I) pour obtenir le composé de formule (II) suivante ou l'un de ses sels,

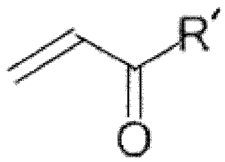

Formule (II)

où R' représente un groupe COOR1 ou $CH_2OR2$ pour lequel R1 et R2, identiques ou différents, représentent un groupe choisi parmi les groupes alkyle, linéaires ou ramifiés, ayant de 1 à 12 atomes de carbone, et les groupes cycloalkyle ayant de 3 à 12 atomes de carbone,
et, on fait réagir ledit composé (I) ou ledit composé (II) ou lesdits un de leurs sels, avec le méthylmercaptan pour obtenir ledit composé (III) ou ledit un de ses sels, au moins.

**11.** Procédé selon la revendication 10, caractérisé en ce l'oxydation est réalisée selon la revendication 8 et le composé (III) est le l-hydroxy-4-méthylthiobutan-2-one.

**Patentansprüche**

**1.** Verfahren zur Synthese von mindestens einer Verbindung der folgenden Formel (I) oder eines ihrer Salze,

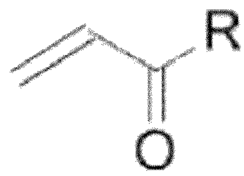

Formel (I),

in der R für eine COOH-, $CH_2OH$- oder CHO-Gruppe steht,
<u>**dadurch gekennzeichnet, dass**</u>
das But-3-en-1,2-diol (BDO) einer Oxidation in Gegenwart eines Katalysators unterzogen wird, wobei der Katalysator eine aktive Phase auf der Basis von mindestens einem Edelmetall, das aus Palladium, Gold, Silber, Platin, Rhodium, Osmium, Ruthenium und Iridium ausgewählt ist, und einen Träger, der basische Stellen enthält und aus Hydrotalkiten (HT), Bruciten, Hydroxyapatit $Ca_{10}(PO_4)_6(OH)_2$, Tricalciumphosphat $Ca_3(PO_4)_2$, Calciumhydrogenphosphat $CaHPO_4(0\text{-}2)H_2O$, Calciumdiphosphat $Ca_2P_2O_7$, Octacalciumphosphat $Ca_8H_2(PO_4)_6.5H_2O$, Tetracalciumphosphat $Ca_4(PO_4)_2O$, amorphen Calciumphosphaten $Ca_3(PO_4)_2\cdot nH_2O$, Calcium-, Cäsium-, Lithium-, Rubidium-, Kalium-, Magnesium-, Barium-, Cer-, Lanthan-, Aluminium-, Zink-, Kupfer-Phosphaten, Diphosphaten und Hydrogenphosphaten, und deren Gemischen ausgewählt ist, umfasst.

2. Verfahren nach Anspruch 1, **dadurch gekennzeichnet, dass** die aktive Phase aus Palladium oder aus einem Gemisch von Palladium und mindestens einem Edelmetall, das aus Platin und Gold ausgewählt ist, besteht.

3. Verfahren nach Anspruch 1 oder 2, **dadurch gekennzeichnet, dass** die aktive Phase aus einem Edelmetall oder einem Gemisch von Edelmetallen in einem Gehalt, der von 0,005 bis 50 Gew.-% reicht, bezogen auf das Gewicht des Trägers in Oxidform, besteht.

4. Verfahren nach Anspruch 1, **dadurch gekennzeichnet, dass** der Träger aus Verbindungen gemäß den folgenden Formeln A, B und C und deren Gemischen ausgewählt ist:

Formel (A), $M_{\alpha}[Al_{(1-b)}La_{b}]A^{z-}]_{c}$,

in der

M aus der Gruppe bestehend aus $Mg^{2+}$, $Ca^{2+}$, $Sr^{2+}$, $Ba^{2+}$, $Ra^{2+}$ und deren Kombinationen ausgewählt ist,
$A^{z-}$ ein einwertiges oder zweiwertiges Anion, das aus der Gruppe bestehend aus Carbonat ($CO_3^{2-}$, in dem die Ladung "z" durch z = 2 gegeben ist), Oxid ($O^{2-}$, in dem z = 2), Hydroxid (OH-, in dem z = 1) und Bicarbonat ($HCO_3^-$, in dem z = 1) ausgewählt ist, oder ein Gemisch ($A^{z'-}{}_x A^{z''-}{}_y$) von zweiwertigen und einwertigen Anionen ist, wobei $A^{z'-}$ und $A^{z''-}$ unterschiedliche Anionen sind, wobei $A^{z'-}A^{z''-}{}_x A^{z''-}{}_y$ ist, mit einer Ladung z, die durch z = x(z') + y(z") gegeben ist, und x + y = 1,
$\alpha$ von 0,01 bis 0,4 variiert,
b von 0,0011 bis 0,11 variiert,

$$c = (2\alpha / z) + [3(1 - b) / z] + (3b / z),$$

Formel (B): $(M_d M'_e M''_f M'''_g)_5(PO_4)_3(OH)$,

in der

M $Mg^{2+}$ ist; M' $Ca^{2+}$ ist; M" $Sr^{2+}$ ist, M'" $Ba^{2+}$ ist,
d von 0 bis 1 variiert,
e von 0 bis 0,5 variiert,
f von 0 bis 1 variiert,
g von 0 bis 1 variiert,
d + e + f + g = 1,

Formel (C), $(M_dM'_eM''_fM'''_g)_3(PO_4)_2$,

in der

M $Mg^{2+}$ ist; M' $Ca^{2+}$ ist; M" $Sr^{2+}$ ist, M'" $Ba^{2+}$ ist,
d von 0 bis 1 variiert,
e von 0 bis 1 variiert,
f von 0 bis 1 variiert,
g von 0 bis 1 variiert,
d + e + f + g = 1.

**5.** Verfahren nach einem der Ansprüche 1 bis 4, **dadurch gekennzeichnet, dass** der Katalysator einen Promotor umfasst, der aus Wismut, Blei, Antimon, Zinn, Niob, Tellur, Indium, Gallium, Zink, Kupfer, Nickel, Kobalt, Silber, Wolfram, Molybdän, Zirkon, Vanadium, Chrom, Mangan, Eisen, Cer, Praseodym, Samarium, Titan und deren Gemischen ausgewählt ist.

**6.** Verfahren nach einem der Ansprüche 1 bis 5, **dadurch gekennzeichnet, dass** der Gehalt des Promotors des Katalysators von 0,005 bis 500 Gew.-% reicht, vorzugsweise zwischen 0,005 und 100 Gew.-% liegt, bezogen auf das Gewicht des Trägers in Oxidform.

**7.** Verfahren nach einem der Ansprüche 1 bis 6, **dadurch gekennzeichnet, dass** das BDO in wässriger Lösung in einer Konzentration ist, die von 1 bis 70 Gew.-% reicht, bezogen auf das Gewicht der Lösung.

**8.** Verfahren nach einem der Ansprüche 1 bis 7, **dadurch gekennzeichnet, dass** die Oxidation in Gegenwart eines Katalysators durchgeführt wird, dessen aktive Phase aus Palladium und Gemischen von Palladium und Platin ausgewählt ist, und der Träger mit basischen Stellen aus Hydroxyapatit und Hydrotalkit ausgewählt ist und die Verbindung (I) Vinylcetylalkohol (VCA) ist.

**9.** Verfahren zur Synthese von mindestens einer Verbindung der folgenden Formel (II) oder eines ihrer Salze,

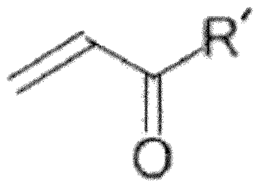

Formel (II),

in der R' für eine COOR1 - oder $CH_2OR2$-Gruppe steht, wobei R1 und R2, die gleich oder verschieden sind, für eine Gruppe stehen, die aus linearen oder verzweigten Alkylgruppen mit 1 bis 12 Kohlenstoffatomen und Cycloalkylgruppen mit 3 bis 12 Kohlenstoffatomen ausgewählt sind,

<u>**dadurch gekennzeichnet, dass**</u>

das But-3-en-1,2-diol (BDO) einer Oxidation in Gegenwart eines Katalysators nach einem der Ansprüche 1 bis 8 unterzogen wird, um eine Verbindung der folgenden Formel (I) zu erhalten,

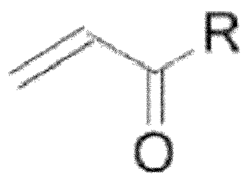

Formel (I),

in der R für eine COOH-, $CH_2OH$- oder CHO-Gruppe steht, nach einem in einem der Ansprüche 1 bis 8 definierten

Verfahren, und
die Veresterung oder Veretherung der Verbindung der Formel (I) durchgeführt wird, um die Verbindung der Formel (II) zu erhalten.

**10.** Verfahren zur Synthese von mindestens einer Verbindung der folgenden Formel (III) oder eines ihrer Salze,

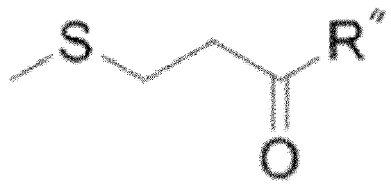

Formel (III),

in der R" für eine COOH-, COOR1-, $CH_2OH$-, $CH_2OR2$- oder CHO-Gruppe steht, wobei R1 und R2, die gleich oder verschieden sind, für eine lineare oder verzweigte Alkylgruppe mit 1 bis 12 Kohlenstoffatomen und Cycloalkylgruppen mit 3 bis 12 Kohlenstoffatomen stehen,
**dadurch gekennzeichnet, dass**
das But-3-en-1,2-diol (BDO) einer Oxidation in Gegenwart eines Katalysators unterzogen wird, um mindestens eine Verbindung der folgenden Formel (I) oder eines ihrer Salze zu erhalten,

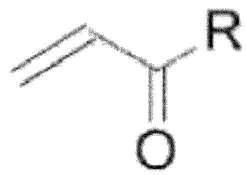

Formel (I),

in der R für eine COOH-, $CH_2OH$- oder CHO-Gruppe steht, nach einem in einem der Ansprüche 1 bis 8 definierten Verfahren,
wenn R" für eine COOR1- oder $CH_2OR2$-Gruppe steht, die Veresterung oder Veretherung der Verbindung der Formel (I) durchgeführt wird, um die Verbindung der folgenden Formel (II) oder eines ihrer Salze zu erhalten,

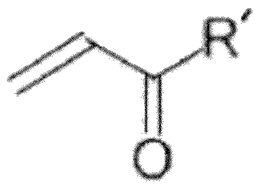

Formel (II),

in der R' für eine COOR1 - oder $CH_2OR2$-Gruppe steht, wobei R1 und R2, die gleich oder verschieden sind, für eine Gruppe stehen, die aus linearen oder verzweigten Alkylgruppen mit 1 bis 12 Kohlenstoffatomen und Cycloalkylgruppen mit 3 bis 12 Kohlenstoffatomen ausgewählt sind,
und die Verbindung (I) oder die Verbindung (II) oder das eine ihrer Salze mit Methylmercaptan reagieren gelassen wird, um die Verbindung (III) oder das eine ihrer Salze zu erhalten.

**11.** Verfahren nach Anspruch 10, **dadurch gekennzeichnet, dass** die Oxidation nach Anspruch 8 durchgeführt wird und die Verbindung (III) 1-Hydroxy-4-methylthiobutan-2-on ist.

## Claims

1. A method for synthesizing at least one compound of the following formula (I) or one of the salts thereof,

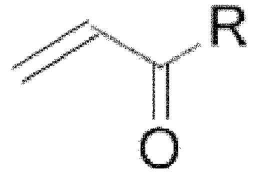

Formula (I)

where R represents a COOH, $CH_2OH$ or CHO group,
**Characterized in that**
but-3-ene-1,2-diol (BDO) is subjected to oxidation in the presence of a catalyst, said catalyst comprising an active phase based on at least one noble metal selected from palladium, gold, silver, platinum, rhodium, osmium, ruthenium and iridium, and a support containing base sites and selected from hydrotalcites (HT), brucites, hydroxyapatite $Ca_{10}(PO_4)_6(OH)_2$, tricalcium phosphate $Ca_3(PO_4)_2$, calcium hydrogenphosphate $CaHPO_4(0\text{-}2)H_2O$, calcium diphosphate $Ca_2P_2O_7$, octacalcium phosphate $Ca_8H_2(PO_4)_6.5H_2O$, tetracalcium phosphate $Ca_4(PO_4)_2O$, amorphous calcium phosphates $Ca_3(PO_4)_2.nH_2O$, phosphates, diphosphates, and hydrogenphosphates of calcium, cesium, lithium, rubidium, potassium, magnesium, barium, cerium, lanthanum, aluminum, zinc, copper, and mixtures thereof.

2. The method according to claim 1, **characterized in that** the active phase consists of palladium or a mixture of palladium and at least one noble metal selected from platinum and gold.

3. The method according to claim 1 or 2, **characterized in that** the active phase consists of a noble metal or a mixture of noble metals in a content ranging from 0.005 to 50% by weight relative to the weight of the support in the oxide form.

4. The method according to claim 1, **characterized in that** the support is selected from the compounds having the following formulas A, B and C, and mixtures thereof:

Formula (A), $M_{\alpha}[Al_{(1-b)}La_{b}]A^{z-}]_{c}$

where

M is selected from the group composed of $Mg^{2+}$, $Ca^{2+}$, $Sr^{2+}$, $Ba^{2+}$, $Ra^{2+}$, and the combinations thereof,
$A^{z-}$ is a monovalent or divalent anion selected from the group composed of carbonate ($CO_3^{2-}$, where the charge « z » is given by z=2), oxide ($O^{2-}$, where z=2), hydroxide ($OH^-$, where z=1), and bicarbonate ($HCO_3^-$, where z=1) or a mixture ($A^{z'-}_x$, $A^{z''-}_y$) of divalent and monovalent anions with $A^{z'-}$ and $A^{z''-}$ being different anions, with $A^{z'-}$ is $A^{z'-}_xA^{z''-}_y$ with a charge z given by z=x(z')+y(z") and x+y=1
$\alpha$ ranges from 0.01 to 0.4
b ranges from 0.0011 to 0.11

$$c=(2\alpha/z)+[3(1-b)/z]+(3b/z)$$

Formula (B): $(M_dM'_eM''_fM'''_g)_5(PO_4)_3(OH)$

where

M is $Mg^{2+}$; M' is $Ca^{2+}$; M" is $Sr^{2+}$; M'" is $Ba^{2+}$
d ranges from 0 to 1
e ranges from 0 to 0.5
f ranges from 0 to 1
g ranges from 0 to 1

d+e+f+g=1

Formula (C): $(M_dM'_eM''_fM'''_g)_3(PO_4)_3$

where

M is $Mg^{2+}$; M' is $Ca^{2+}$; M" is $Sr^{2+}$; M'" is $Ba^{2+}$
d ranges from 0 to 1
e ranges from 0 to 1
f ranges from 0 to 1
g ranges from 0 to 1
d+e+f+g=1.

5. The method according to any one of claims 1 to 4, **characterized in that** the catalyst comprises a promoter selected from bismuth, lead, antimony, tin, niobium, tellurium, indium, gallium, zinc, copper, nickel, cobalt, silver, tungsten, molybdenum, zirconium, vanadium, chromium, manganese, iron, cerium, praseodymium, samarium, titanium and mixtures thereof.

6. The method according to any one of claims 1 to 5, **characterized in that** the content of the promoter of the catalyst ranges from 0.005 to 500%, preferably between 0.005 and 100%, by weight relative to the weight of the support in the oxide form.

7. The method according to any one of claims 1 to 6, **characterized in that** the BDO is in aqueous solution, in a concentration ranging from 1 to 70% by weight relative to the weight of the solution.

8. The method according to any one of claims 1 to 7, **characterized in that** the oxidation is carried out in the presence of a catalyst whose active phase is selected from palladium and the mixtures of palladium and platinum and the base site support is selected from hydroxyapatite and hydrotalcite, and the compound (I) is vinyl keto alcohol (CALV).

9. The method for synthesizing at least one compound of the following formula (II) or one of the salts thereof,

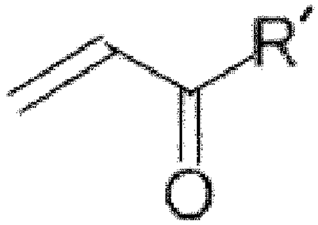

Formula (II)

where R' represents a COOR1 or $CH_2OR2$ group for which R1 and R2, identical or different, represent a group selected from the linear or branched alkyl groups having 1 to 12 carbon atoms, and the cycloalkyl groups having 3 to 12 carbon atoms,

**characterized in that**

but-3-ene-1,2-diol (BDO) is subjected to oxidation in the presence of a catalyst, according to any one of claims 1 to 8, to obtain a compound of the following formula (I),

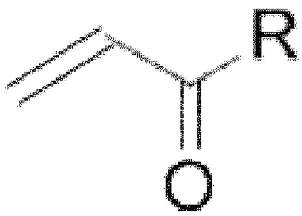

Formula (I)

where R represents a COOH, $CH_2OH$ or CHO group, according to a method defined in any one of claims 1 to 8, and
the esterification or the etherification of the compound of formula (I) is carried out to obtain the compound of formula (II).

**10.** The method for synthesizing at least one compound of the following formula (III) or one of the salts thereof,

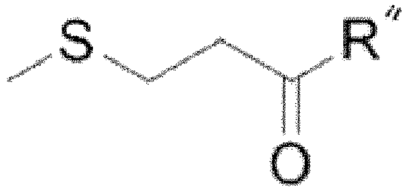

Formula (III)

where R" represents a COOH, COOR1, $CH_2OH$, $CH_2OR2$ or CHO group for which R1 and R2, identical or different, represent a linear or branched alkyl group, having 1 to 12 carbon atoms, and the cycloalkyl groups having 3 to 12 carbon atoms,
**characterized in that**
but-3-ene-1,2-diol (BDO) is subjected to oxidation in the presence of a catalyst to obtain at least one compound of the following formula (I) or one of the salts thereof,

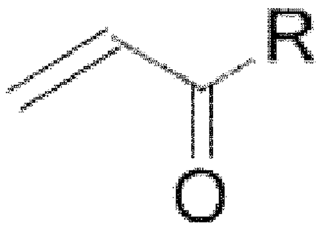

Formula (I)

where R represents a COOH, $CH_2OH$ or CHO group, according to a method defined in any one of claims 1 to 8,
if R" represents a COOR1 or $CH_2OR2$ group, the esterification or etherification of the compound of formula (I) is carried out to obtain the compound of the following formula (II) or one of the salts thereof,

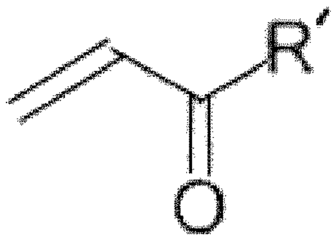

Formula (II)

where R' represents a COOR1 or $CH_2OR2$ group for which R1 and R2, identical or different, represent a group selected from the linear or branched alkyl groups, having 1 to 12 carbon atoms, and the cycloalkyl groups having 3 to 12 carbon atoms,
and, said compound (I) or said compound (II) or said one of the salts thereof is reacted with methylmercaptan to obtain said compound (III) or said one of the salts thereof, at least.

**11.** The method according to claim 10, **characterized in that** the oxidation is carried out according to claim 8 and the compound (III) is 1-hydroxy-4-methylthiobutan-2-one.

FIGURE 1

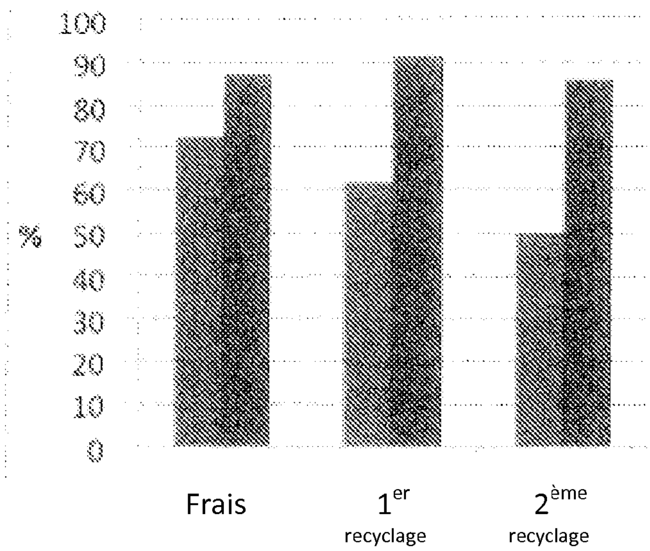


FIGURE 2

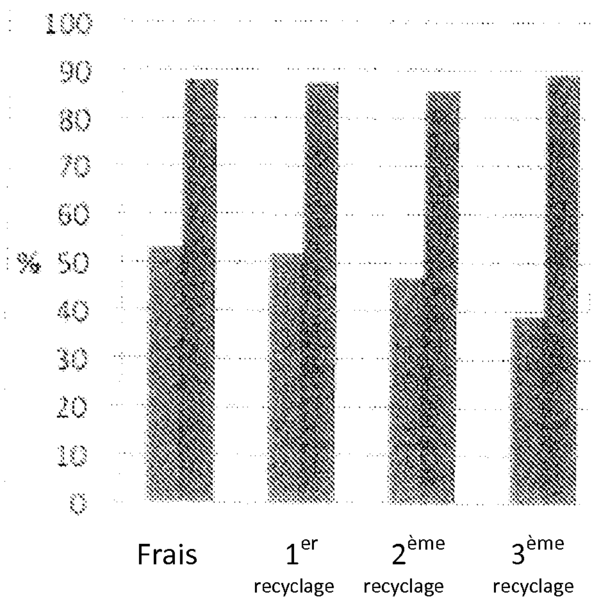

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2880345 A1
- US 2004204597 A1


### Littérature non-brevet citée dans la description


- **K. MORI ; T. HARA ; T. MIZUGAKI ; K. EBITANI ; K. KANEDA.** *J. Am. Chem. Soc.,* 2004, vol. 126 (34), 10657-10666
- **N. K. GUPTA ; S. NISHIMURA ; A. TAKAGAKI ; K. EBITANI.** *Green Chem.,* 2011, vol. 13, 824-827